Europäisches Patentamt

(19) European Patent Office

Office européen des brevets

(11) Publication number: **0 311 590**
**A2**

## EUROPEAN PATENT APPLICATION

(21) Application number: **88870157.0**

(22) Date of filing: **07.10.88**

(51) Int. Cl.⁴: **A 61 K 35/12**
**A 23 L 1/29**

(30) Priority: **08.10.87 US 106679**

(43) Date of publication of application:
**12.04.89 Bulletin 89/15**

(84) Designated Contracting States:
**AT BE CH DE FR GB IT LI LU NL SE**

(71) Applicant: **Lefkowith, James Bruce**
**7920 Cornell Avenue**
**St. Louis Missouri 63130 (US)**

**Schreiner, George Frederic**
**4624 Pershing Place**
**St. Louis Missouri 63108 (US)**

(72) Inventor: **Lefkowith, James Bruce**
**7920 Cornell Avenue**
**St. Louis Missouri 63130 (US)**

**Schreiner, George Frederic**
**4624 Pershing Place**
**St. Louis Missouri 63108 (US)**

(74) Representative: **Lunt, John Cooper et al**
**Monsanto Europe S.A. Patent Department Avenue de**
**Tervuren 270-272 Letter Box No 1**
**B-1150 Brussels (BE)**

(54) Method of improving the suitability of mammalian organs and tissue for transplantation.

(57) Mammalian organs or tissue having improved suitability for transplantation are obtained by subjecting a mammal to an essential fatty acid deficient diet, and removing the organ or tissue from the mammal. The organ or tissue can be implanted in a recipient without the need for the administration of immunosuppressants to the recipient.

EP 0 311 590 A2

**Description**

**Method of improving the suitability of mammalian organs and tissue for transplantation**

This invention relates to a method of obtaining a mammalian organ or tissue having improved suitability for transplantation and, more particularly, to a method of reducing allograft rejection in organ and tissue transplants.

The treatment of patients with end-stage renal disease has evolved into an important branch of medical therapeutics. Today, many thousands of patients are on chronic haemodialysis. Many of these patients are awaiting renal transplantation.

A major problem that exists with kidney transplantation, as well as with the transplantation of other organs and tissues such as the heart, lung, liver, and pancreas, is the so-called rejection mechanism. This problem is particularly acute in the case of incomplete tissue crossmatch compatibility. Typically, immunosuppressive drugs are administered to the transplant recipient to reduce the likelihood of rejection.

The discovery of the profound effect of cyclosporin A on the immune system, mediated by its non-lethal suppression of T-lymphocytes, has enabled its clinical use in organ transplantation. The cyclosporin A suppresses the recipient's cellular immune response, thereby preventing acute renal allograft rejection. See, e.g., Merion et al, New England. J. Med. 310, 148-154 (1984). However, cyclosporin A does have side effects. A major side effect is that it carries the danger of toxicity, especially to the kidneys and liver. That is, elevated levels of oreatinine are found in the blood of many renal transplant patients treated with cyclosporin A. Some minor side effects are also evident with the immunosuppressive treatment. Therefore, the administration of cyclosporin A must be carefully monitored.

Accordingly, a method of transplantation which does not require immunosuppression would have significant importance to medical therapeutics.

In accordance with the present invention, a method is provided for obtaining a mammalian organ or tissue having improved suitability for transplantation, which comprises subjecting a mammal to an essential fatty acid deficient diet and removing the organ or tissue from the mammal. A mammal which has been subjected to such a diet is referred to as a mammal having essential fatty acid deficiency (EFAD). Organs or tissue thus obtained can be transplanted into a recipient without administration of immunosuppressive drugs to the recipient.

As used herein, the biochemical definition of EFAD is that described by Holman, J. Nutrition 70, 405-410 (1960). That is, EFAD is the state in which the 20:3 (n-9) to 20:4 (n-6) ratio in hepatic phospholipids exceeds 0.4. The 20:3 (n-9) fatty acid is also known as Mead acid, Fulco and Mead, J. Biol. Chem 234, 1411-1416 (1959). The 20:4 (n-6) is commonly known as arachidonic acid.

A preferred mammalian organ for use in the method of the invention is a kidney obtained from an EFAD mammal, although other organs and tissue such as, for example, pancreatic islets and cardiac tissue, can also be used.

A central hypothesis in transplantation biology is that resident leukocytes expressing class 11 histocompatibility antigens may determine the immunogenicity of an organ (Lacy, Clin. Chem. 32, B76-B82 (1982)). Using the novel method of the present invention, EFAD kidneys, which were depleted of resident la-positive macrophages, exhibited sustained survival and function when transplanted across a major histocompatibility antigen barrier in the absence of immunosuppression of the recipient. By way of comparison, untreated control allografts of normal kidneys all rejected promptly.

By co-administration of la-positive cells at the time of transplantation, it was established that the lack of resident leukocytes due to EFAD was responsible for the protective effect, as opposed to the lipid alterations of EFAD per se.

Detailed Description of the Invention

While the specification concludes with claims particularly pointing out and distinctly claiming the subject matter regarded as forming the present invention, it is believed that the invention will be better understood from the following description taken in conjunction with the accompanying drawings in which briefly:

FIG. 1 is a graphical representation or bar chart which shows the glomerular and renal interstitial macrophage (Mφ) content in EFAD versus control organs.

FIG. 2 is a graphical representation which shows the lipid and macrophage depletion of EFAD allografts in days post transplant.

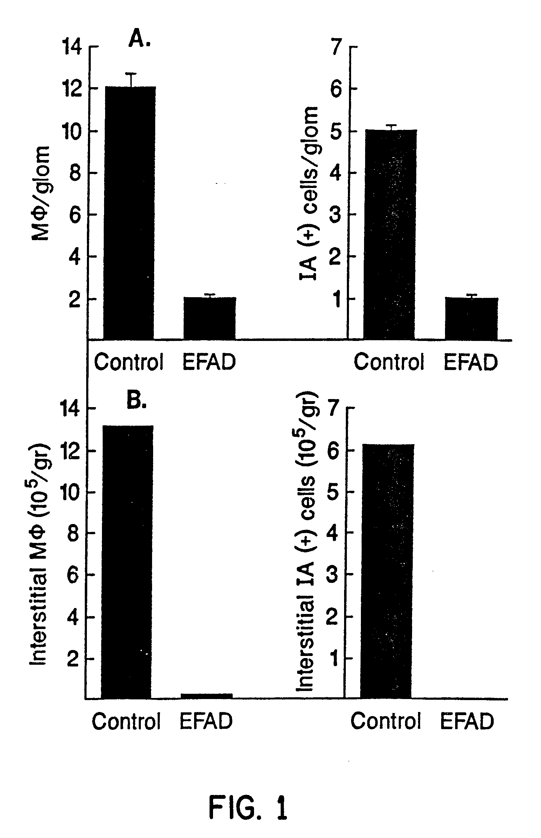

In a series of tests, Lewis rats were placed on an EFAD diet for a minimum of two months prior to kidney donation. As shown in Table I, below, both hepatic and renal phospholipids exhibited the characteristic changes of the deficiency state. All phospholipids manifested a depletion of arachidonate and an accumulation of 20:3 (n-9), or Mead acid, the fatty acid which uniquely accumulates in EFAD. [Fulco and Mead, J. Biol. Cham. 234, 1411-1416 (1959)]. Phosphatidylcholine and phosphatidylinositiol were typically the most significantly affected phospholipids. Figure 1 illustrates the macrophage content and la-positive cell content of these kidneys. The depletion of glomerular macrophages and their la-positive subset exceeded 80% in all sampled animals through the course of the test. The effects on the leukocyte content of the interstitium were even more marked, with nearly complete elimination of interstitial macrophages and Ia-positive cells from the EFAD kidneys.

In order to determine whether the depletion of resident macrophages diminished the allogeneic potential of the kidney, kidneys from Lewis EFAD donors (RT11) were subsequently transplanted to Buffalo recipients (RT1b) differing at the major histocompatibility locus. The recipients received no immunosuppressive therapy prior to, or subsequent to, transplantation of the renal allograft and were maintained on a normal diet throughout the test. As shown in Table II, below, all control Lewis kidneys transplanted to Buffalo recipients experienced severe and irreversible cellular rejection by day 5 as judged by histological criteria. In contrast, none of the EFAD donor kidneys exhibited any evidence of cellular infiltrate for up to two weeks post-transplantation. Ten recipients of EFAD allografts had their remaining contralateral kidney removed at 1 month. These EFAD allografts exhibited adequate enough function to sustain the recipient on the graft alone for up to five months.

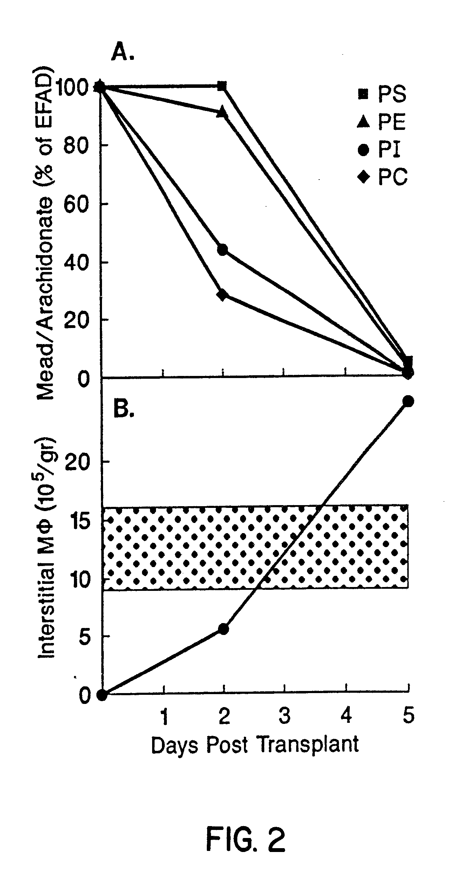

The kinetics of lipid repletion and macrophage repopulation of the allograft are shown in Figure 2. Mead acid rapidly disappeared from the allograft because of the normal lipid composition of the recipient animal. Substantial normalization of lipids was observed two days after transplantation and was essentially complete by day 5. Over this same time period, the content of interstitial macrophages returned to a level slightly above normal. These cells expressed the class I major histocompatibility antigens of the Buffalo recipient (data not shown) and appeared to represent a re-stocking phenomenon. The kinetics of lipid repletion and macrophage repopulation of the allograft appear to correlate. In contrast to the restocking of interstitial macrophages observed in EFAD Lewis allografts, rejecting normal Lewis allografts had a greater than 15-fold increase in interstitial macrophages (13.0 to 217.4 x $10^5$ cells/gram tissue), reflecting the rejection infiltrate.

The abrogation of rejection of the EFAD allografts in the absence of immunosuppression could not be attributed to the transfer of an immunosuppressive agent within the EFAD kidney. When Lewis Ia-positive leukocytes were injected intraperitoneally into the recipients at the time of the EFAD kidney transplant, the kidneys manifested histological evidence of rejection on day 5 (Table II). Splenocytes from EFAD or control Lewis rats were equally effective in inducing rejection of the kidney allograft. Thus, if the organ macrophage depletion induced by the EFAD state was reversed by the provision of Ia-positive macrophages at the time of transplantation, rejection of the allograft ensued.

The following detailed examples illustrate the uniformly successful transplantation of a kidney across a major histocompatibility barrier in the absence of any immunosuppression. The effect appears directly attributable to depletion of resident Ia-positive leukocytes, a depletion that occurs as a result of EFAD in the donor animal. The lack of a requirement for any immunosuppression is notable in the rat in that prior tests involving in vitro leukocyte depletion of pancreatic islets have required some degree of subsequent immunosuppression of the recipient [Lacy, Clin. Chem. 32, B76-B82 (1986)].

There are prior reports of prolonged renal and cardiac allograft survival as a result of resident Ia-positive cell depletion of the allograft [McKenzie et al., Transplant. Proc. 16, 948-951 (1984) and Transplantation 38, 371-376 (1984)]. Depletion in these reports was accomplished with a combination of lethal irradiation and cyclophosphamide treatment of the donor. This regimen, however, was not invariably successful in either depleting resident Ia-positive cells or in preventing histologic evidence of rejection. In contrast, the present inventors have observed leukocyte depletion with EFAD in all strains of rat tested including Lewis, Buffalo, and Sprague-Dawley, and have observed no infiltrate in any allograft.

The examples also illustrate the depletion of Ia-positive cells in pancreatic islets and cardiac tissue with EFAD.

It will be appreciated that the invention is not limited to the following Examples which are given by way of illustration.

Example 1

Rats were made EFAD by feeding them a fat-free diet (#5803 EFA Low Diet, Purina Test Diets, Richmond, IN) for at least 8 weeks. Control rats were fed a standard laboratory diet (#5755 Basal Diet, Purina Test Diets, Richmond, IN). The standard laboratory diet provided about 19% protein, 10% fat, 3% fiber, 61% carbohydrate and the balance of nutrient vitamins and minerals. The fat-free diet was similar except for the absence of the fat which was replaced with carbohydrate. Liver and kidney samples from donor animals were removed at the time of donation for fatty acid analysis. Hepatic and renal phospholipids were extracted using a Bligh-Dyer extraction, and separated by two dimensional thin layer chromatography as described previously by Lefkowith et al., J. Biol. Chem. 260, 15736-15744 (1985). The fatty acids within each phospholipid were then transmethylated, and the resultant fatty acid methyl esters characterized by gas chromatography also as detailed previously by Lefkowith et al., supra. The changes in the major (n-6) and n-9 fatty acids with EFAD are shown in Table I, below. Results are expressed as mol% (mean ± standard error, n = 3-5). With EFAD, tissue (n-9) fatty acids rose and (n-6) fatty acids fell. In particular 20:3 (n-9) was detected whereas this fatty acid was not present normally. The 20:3 (n-9) to 20:4 (n-6) ratio in all hepatic phospholipids vastly exceeded 0.4 which is the biochemical definition of EFAD [Holman, J. Nutrition 70, 405-410 (1960)]. Renal lipids, in contrast, exhibited more modest changes as has been described before by Lefkowith et al., supra. Abbreviations: PC, phosphatidylcholine; PE, phosphatidylethanolamine; PI, phosphatidylinositol; PS, phosphatidylserine; N.D.,

none detected.

EP 0 311 590 A2

Table I

Donor Hepatic and Renal Phospholipid Fatty Acid Composition

| | 18:1(n-9) | | 18:2(n-6) | | 20:3(n-9) | | 20:4(n-6) | |
| | Control | EFAD | Control | EFAD | Control | EFAD | Control | EFAD |
|---|---|---|---|---|---|---|---|---|
| **Hepatic Phospholipids** | | | | | | | | |
| PC | 5.2±0.2 | 24.7±1.5 | 9.8±0.5 | 1.5±0.3 | N.D. | 16.6±1.3 | 33.0±0.9 | 4.5±0.7 |
| PE | 4.6±0.2 | 16.5±1.5 | 5.0±0.5 | 0.5±0.2 | N.D. | 19.0±0.9 | 28.7±0.6 | 15.3±1.7 |
| PI | 1.8±0.6 | 6.0±0.5 | 0.7±0.3 | 0.3±0.3 | N.D. | 39.0±0.6 | 48.4±0.7 | 2.9±0.3 |
| PS | 4.7±0.3 | 9.6±0.6 | 1.2±0.5 | 1.9±1.6 | N.D. | 11.2±0.9 | 23.4±0.9 | 15.4±1.5 |
| **Renal Phospholipids** | | | | | | | | |
| PC | 11.5±1.3 | 32.3±0.4 | 14.9±1.0 | 2.0±0.0 | N.D. | 10.5±0.9 | 26.5±3.7 | 9.5±1.2 |
| PE | 6.6±1.6 | 17.2±0.4 | 2.8±1.1 | 0.8±0.2 | N.D. | 10.8±0.8 | 53.9±7.1 | 33.3±1.6 |
| PI | 6.7±0.6 | 13.9±0.3 | 3.1±0.8 | 0.2±0.2 | N.D. | 23.6±0.7 | 37.9±1.9 | 13.9±0.7 |
| PS | 9.6±2.0 | 12.1±0.3 | 2.7±1.2 | N.D. | N.D. | 6.7±0.1 | 33.1±6.3 | 31.9±2.9 |

Example 2

Kidneys were harvested from Lewis rat donors (both EFAD and normal animals from Example 1) after thorough perfusion with saline containing papaverine 0.5 mg/ml. Papaverine addition was necessary to effect complete wash out of circulating leukocytes. Buffalo rats on a normal diet were used as the recipients. These animals were anesthetized and the left kidney was surgically removed. The Lewis allograft was then placed in the vacant left renal bed and anastomosed to the aorta and residual renal vein, the latter attachment using a previously described cuff technique [Kamada, Transplantation 39, 93-95 (1985)]. The native right kidney was left in situ. A small percentage of EFAD and control grafts failed due to ischemia (17% vs. 10% for EFAD and control grafts, respectively) and were not included in the analysis. Three EFAD allografts developed hydronephrosis after 5 days and these animals were also excluded. After 2, 5, and 14 days the allografts were removed to provide tissue for histologic analysis. Tissue specimens were preserved in 10% formalin in phosphate-buffered saline and later sectioned, stained with hematoxylin/eosin, and examined by light microscopy. Rejection was defined by the presence of moderate to severe interstitial nephritis with periglomerular, perivascular and peritubular infiltrates of mononuclear cells. Kidneys were defined as not rejecting when no cellular infiltrate and a normal parenchyma was observed. No intermediate abnormal histology was observed. In several tests with EFAD grafts, recipients were also given an intraperitoneal inoculum of $10^8$ dissociated Lewis spleen cells in concert with the allograft. In 3 animals EFAD spleen cells were given, and in 2 tests control spleen cells were used. Approximately 15% of the spleen cells were Ia-positive. No significant difference between control and EFAD animals with respect to the percentage of spleen cells expressing Ia was seen. - indicates not done. Results are shown in Table II, below.

Table II

Rejection of Control and EFAD Renal Allografts

| | Kidneys rejection/total transplants at each time point | | |
|---|---|---|---|
| | 2 days | 5 days | 14 days |
| Control allografts | 0/2 | 7/7 | - |
| EFAD allografts | 0/4 | 0/7 | 0/10 |
| EFAD allografts + EFAD spleen cells | - | 3/3 | - |
| control spleen cells | - | 2/2 | - |

Example 3

Renal cortex from EFAD kidneys of Example 1 was dissociated into glomeruli and individual interstitial cells by a modification of a method described by Lefkowith and Schreiner, J. Clin. Invest. 80, October (1987), in press.

A) Glomerular labeling for macrophages and Ia-positive cells:

Glomeruli were isolated from saline-perfused kidneys as previously described by Schreiner et al., J. Clin. Invest. 68, 920-931 (1981). Glomeruli were then subjected to mild enzymatic digestion consisting of collagenase type II (Worthington Biochemicals, Freehold, NJ) 500 µg/ml and DNAse (Sigma Chemical Co., St. Louis, MO) 0.1 µg/ml in Hanks balanced salt solution for 30 minutes at 23°C. This procedure leaves glomeruli intact but renders them permeable to antibody [Schreiner et al., supra]. Glomeruli were then labeled with anti-macrophage antiserum or anti-Ia antiserum and then sandwich-labeled with the appropriate FITC-antibody as detailed previously by Schreiner et al., supra. Positively labeled cells within 50 to 100 glomeruli were counted. Results are shown in Figure 1A and expressed as number of cells per glomerulus (glom). The results represent the data from three separate tests (mean ± standard error). Each test used a pool of glomeruli from two animals.

B) Interstitial labeling of macrophages and la-positive cells:

Dissected renal cortex from saline-perfused kidneys was minced through a 250 μ screen and subjected to the same digestion protocol as above save for the addition of soybean trypsin inhibitor (Sigma Chemical Co., St. Louis, MO) 1 mg/ml. This procedure results in a single cell suspension of tubular epithelial and interstitial cells along with intact glomeruli as described by Lefkowith and Schreiner, J. Clin. Invest. 80, October (1987), in press. Interstitial cells were labeled for macrophage antigen and la antigen as above. Positively labeled cells in the interstitial population were counted on a per volume basis and normalized for the weight of the dissected sieved cortex prior to digestion (expressed as $10^5$ cells/gr. cortex). Results are shown in Figure 1B in which the bars reflect the averaged results of two separate tests. Each test used a pool of cortex from two animals.

## Example 4

Tissue samples from EFAD allografts derived from the test described in Example 2 were obtained on days 0, 2, and 5.

## A) EFAD allograft lipid analysis:

The lipids from tissue samples from allografts were extracted, and the 20:3 (n-9) to arachidonate ratio was determined by gas chromatographic analysis as detailed in Example 1. The results are shown in Figure 2A in which the values were relativized by taking the 20:3 (n-9) to arachidonate ratio present on day 0 as 100%.

## B) EFAD allograft macrophage content:

The macrophage content of the interstitium of EFAD allografts was determined in parallel with the lipid analysis. Details of macrophage quantification are provided in Example 3. The abbreviations PS, PE, PI and PC are as defined in Example 1. The results are shown in Figure 2B in which the stippled area represents the range of normal values seen in rats on a standard diet.

## Example 5

A. Pancreatic islets also were depleted of la-positive cells with EFAD. Islets were removed from the pancreas from animals placed on an EFAD diet using a standard protocol [Lacy and Kostianovsky, Diabetes 16, 35-39 (1967)]. These islets were then labelled for la-positive cells in a manner analogous to glomeruli as detailed in Example 3, above. la-positive cells within individual islets were then counted in between 20 and 50 islets using fluorescence microscopy. EFAD islets had 1.2±0.3 la-positive cells per islet as opposed to control islets which had 8.4±1.6 la-positive cells per islet (mean ± standard error).

B. Cardiac tissue also exhibited a depletion of la-positive cells. la-positive cells in heart were enumerated by obtaining frozen sections of tissue, fixing them to glass slides using acetone, and staining these sections using the methods utilized, above, for glomeruli and islets. Between 10 and 20 high power fields were examined per section. EFAD heart tissue had 1.9±0.8 la-positive cells per high power field. Control heart tissue had 4.3±0.9 la-positive cells per high power field.

## Claims

1. A method of obtaining a mammalian organ or tissue having improved suitability for transplantation, which comprises subjecting a mammal to an essential fatty acid deficient diet and removing the organ or tissue from the mammal.

2. A method according to Claim 1 in which the mammal is non-human and the mammal is slaughtered before the organ or tissue is removed.

3. A method according to either of Claims 1 and 2 in which the organ or tissue is a kidney.

4. A method of reducing the number of resident la-positive macrophages in an organ or tissue of a mammal to thereby improve the suitability of said organ or tissue for transplantation, comprising subjecting said mammal to an essential fatty acid deficient diet, removing the organ or tissue and treating the same to substantially remove any remaining leukocytes.

5. A method according to Claim 4 in which the mammal is non-human and the mammal is slaughtered before the organ or tissue is removed.

6. A method according to either of Claims 4 and 5 in which the organ or tissue is a kidney.

7. A method according to either of Claims 4 and 5 in which the organ or tissue is pancreatic islets.

8. A method according to either of Claims 4 and 5 in which the organ or tissue is cardiac tissue.

FIG. 1

FIG. 2